(19) Europäisches Patentamt
European Patent Office
Office européen des brevets

(11) EP 1 097 154 B1

(12) **EUROPÄISCHE PATENTSCHRIFT**

(45) Veröffentlichungstag und Bekanntmachung des Hinweises auf die Patenterteilung:
**01.10.2003 Patentblatt 2003/40**

(21) Anmeldenummer: **99932812.3**

(22) Anmeldetag: **05.07.1999**

(51) Int Cl.[7]: **C07D 491/04**, A61K 31/4184
// (C07D491/04, 319:00), C07D235:00

(86) Internationale Anmeldenummer:
**PCT/EP99/04650**

(87) Internationale Veröffentlichungsnummer:
**WO 00/004022 (27.01.2000 Gazette 2000/04)**

(54) **SUBSTITUIERTE BENZIMIDAZOLE, IHRE HERSTELLUNG UND IHRE VERWENDUNG ALS MITTEL GEGEN PARASITÄRE PROTOZOEN**

SUBSTITUTED BENZIMIDAZOLES, PRODUCTION AND USE THEREOF AS AGENTS FOR COMBATING PARASITIC PROTOZOAS

BENZIMIDAZOLES SUBSTITUES, LEUR PRODUCTION ET LEUR UTILISATION COMME AGENTS POUR COMBATTRE LES PROTOZOAIRES PARASITAIRES

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **16.07.1998 DE 19831985**

(43) Veröffentlichungstag der Anmeldung:
**09.05.2001 Patentblatt 2001/19**

(73) Patentinhaber: **Bayer Aktiengesellschaft**
**51368 Leverkusen (DE)**

(72) Erfinder:
- **GREIF, Gisela**
  **D-53424 Remagen (DE)**
- **HABERKORN, Axel**
  **D-42117 Wuppertal (DE)**
- **BAASNER, Bernd**
  **D-51467 Bergisch-Gladbach (DE)**
- **LIEB, Folker**
  **D-51375 Leverkusen (DE)**
- **MARHOLD, Albrecht**
  **D-51373 Leverkusen (DE)**

(56) Entgegenhaltungen:
**US-A- 5 482 956**

Bemerkungen:
Die Akte enthält technische Angaben, die nach dem Eingang der Anmeldung eingereicht wurden und die nicht in dieser Patentschrift enthalten sind.

EP 1 097 154 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft neue substituierte Benzimidazole, ihre Herstellung und ihre Verwendung als Mittel gegen parasitäre Protozoen.

Die vorliegende Erfindung betrifft ferner Mischungen dieser Verbindungen mit Polyetherantibiotika oder synthetisch hergestellten Coccidiosemitteln in Mitteln zur Bekämpfung parasitärer Protozoen, insbesondere Coccidien.

Substituierte Benzimidazole und ihre Verwendung als Insektizide, Fungizide und Herbizide sind bereits bekannt geworden (EP-OS 87 375, 152 360, 181 826, 239 508, 260 744, 266 984, US-P 3 418 318, 3 472 865, 3 576 818,3 728 994).

Halogenierte Benzimidazole und ihre Wirkung als Anthelmintika, Coccidiostatika und Pestizide sind bekannt geworden (DE-OS 2 047 369, DE-OS 4 237 617). Mischungen von nitrosubstituierten Benzimidazolen und Polyetherantibiotika sind als Coccidiosemittel bekannt geworden (US-P 5 331 003). In allen Fällen befriedigt ihre Wirkung noch nicht.

Coccidiose ist eine Erkrankung, die durch einzellige Parasiten (Protozoen) hervorgerufen wird. Insbesondere bei der Geflügelaufzucht kann sie große Verluste hervorrufen. Um diese zu vermeiden, werden die Bestände prophylaktisch mit Coccidiosemitteln behandelt. Durch die Entwicklung von Resistenzen gegen die eingesetzten Mittel kommt es schon kurz nach Einführung der Mittel zu ernsthaften Problemen. Durch den Einsatz chemisch völlig neuer Coccidiosemittel, insbesondere Kombinationen, ist es andererseits möglich, auch polyresistente Parasitenstämme zu kontrollieren.

Die Benzimidazole der Formel (I)

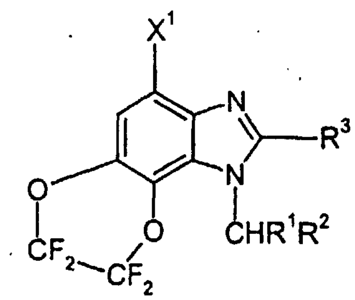

(I),

in welcher

$X^1$ für Chlor oder Brom steht,

$R^1$ Wasserstoff oder $C_{1-4}$-Alkyl steht,

$R^3$ für Fluoralkyl steht,

$R^2$ für den Rest

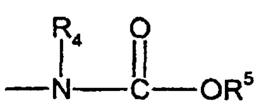

steht,

$R^4$ für Alkyl oder substituiertes Phenyl, das substituiert ist durch $C_{1-4}$-Alkyl, $C_{1-4}$-Halogenalkyl, Halogen, Nitro, $C_{1-4}$-Alkoxy, $C_{1-4}$-Halogenalkoxy, Methylendioxy, Ethylendioxy, die ihrerseits halogensubsituiert sein können, steht,

$R^5$ für Alkyl steht, sind neu und eignen sich besonders zur Bekämpfung parasitärer Protozoen.

Die Benzimidazole der Formel (I)

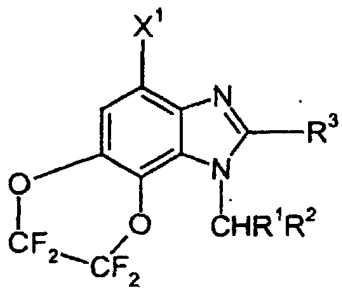

(I),

in welcher

$X^1$ für Chlor oder Brom steht,

$R^1$ Wasserstoff oder $C_{1-4}$-Alkyl steht,

$R^3$ für Fluoralkyl steht,

$R^2$ für den Rest

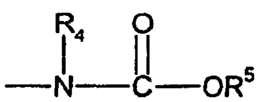

steht,

$R^4$ für Alkyl oder substituiertes Phenyl, das substituiert ist durch $C_{1-4}$-Alkyl, $C_{1-4}$-Halogenalkyl, Halogen, Nitro, $C_{1-4}$-Alkoxy, $C_{1-4}$-Halogenalkoxy, Methylendioxy, Ethylendioxy, die ihrerseits halogensubsituiert sein können, steht,

$R^5$ für Alkyl steht

werden hergestellt, indem man

1 H-Benzimidazole der Formel (II)

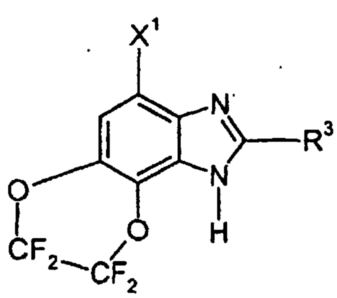

(II),

in welcher

$X^1$ und $R^3$ die oben angegebene Bedeutung haben,

mit einem Alkylierungsmittel der Formel (III)

$$A\text{-}CHR^1R^2 \qquad (III),$$

in welcher

A für eine geeignete Abgangsgruppe steht,

$R^1$ und $R^2$ die oben angegebene Bedeutung haben,

gegebenenfalls in Gegenwart von Verdünnungsmitteln und/oder Reaktionshilfsmitteln umsetzt.

Die Verbindungen der Formel (I) können gegebenenfalls in Abhängigkeit von der Art und Anzahl der Substituenten als geometrische und/oder optische Isomere bzw. Regioisomere oder deren Isomerengemische in unterschiedlicher Zusammensetzung vorliegen. Sowohl die reinen Isomeren als auch die Isomerengemische werden erfindungsgemäß beansprucht.

Die erfindungsgemäßen substituierten Benzimidazole sind durch die Formel (1) allgemein definiert. Bevorzugt sind Verbindungen der Formel (I), bei welchen

$X^1$ für Chlor oder Brom steht,

$R^1$ für Wasserstoff oder $C_{1-4}$-Alkyl wie Methyl, Ethyl, i-Propyl steht,

$R^2$ für den Rest

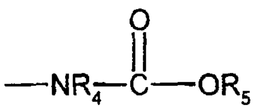

steht,

$R^4$ für $C_{1-6}$-Alkyl wie Methyl, Ethyl, n-Propyl, i-Propyl, n-Butyl, s-Butyl, i-Butyl, t-Butyl, n-Pentyl, n-Hexyl, Cyclohexyl, sowie für substituiertes Phenyl steht. Als Substituenten des Phenylrings kommen dabei in Frage: $C_{1-4}$-Alkyl, insbesondere Methyl, $C_{1-4}$-Halogenalkyl, insbesondere Trifluormethyl, Halogen, Nitro, $C_{1-4}$-Alkoxy, insbesondere Methoxy, Methylendioxy, $C_{1-4}$-Halogenalkoxy, insbesondere Trifluormethoxy, Methylendioxy, Ethylendioxy, die ihrerseits halogensubstituiert sein können,

$R^5$ für $C_{1-4}$-Alkyl, insbesondere Methyl oder Ethyl steht,

$R^3$ für Perfluor- $C_{1-4}$-alkyl, insbesondere Trifluormethyl steht.

Besonders bevorzugt sind Verbindungen der Formel (I), in welcher

$X^1$ für Chlor oder Brom steht,

$R^1$ für Wasserstoff steht,

$R^2$ für den Rest

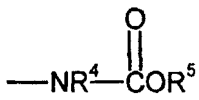

steht,

$R^4$ für C $_{1-6}$-Alkyl steht,

$R^5$ fiir Methyl oder Ethyl steht,

$R^3$ für Trifluormethyl steht.

Verwendet man zur Herstellung der erfindungsgemäßen Verbindungen der Formel (I) beispielsweise Benzimidazol, so läßt sich der Reaktionsablauf des Herstellungsverfahrens durch das folgende Formelschema darstellen:

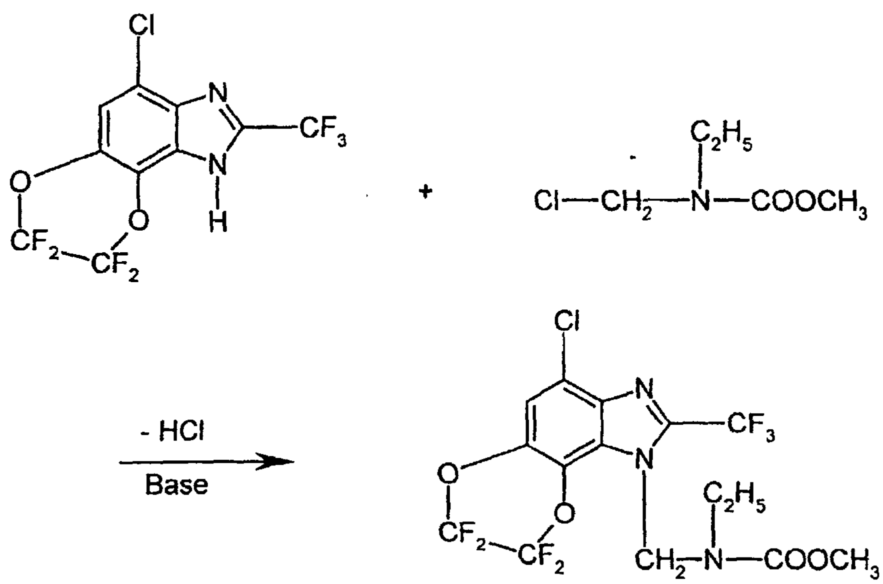


Die zur Durchführung des Herstellungsverfahrens als Ausgangsstoffe benötigten IH-Benzimidazole sind durch die Formel (II) allgemein definiert. In dieser Formel (II) stehen $X^1$ und $R^3$ vorzugsweise für diejenigen Reste, die bereits im Zusammenhang mit der Beschreibung der erfindungsgemäßen Verbindungen der Formel (I) als bevorzugt für diese Substituenten genannt wurden.

Die 1H-Benzimidazole der Formel (II) sind bekannt oder erhältlich in Analogie zu bekannten Verfahren (vergl. z.B. J. Amer. Chem. Soc. 75, 1292 [1953] US-Patent 3,576,818).

Die zur Durchführung des Herstellungsverfahrens weiterhin als Ausgangsprodukte erforderlichen Alkylierungsmittel sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) als bevorzugt für diesen Substituenten genannt wurden.

A steht für einen bei Alkylierungsmitteln üblichen Abgangsrest, vorzugsweise für Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

A steht außerdem auch für eine Hydroxy-, Alkanoyloxy- oder Alkoxygruppe, wie beispielsweise eine Acetoxy- oder Methoxygruppe, insbesondere wenn mit Hilfe des erfindungsgemäßen Verfahrens Verbindungen der Formel (I),

bei welchen $R^1$ verschieden von Wasserstoffist, hergestellt werden sollen.

Die Verbindungen der Formel (III) sind allgemein bekannt oder erhältlich in Analogie zu bekannten Verfahren (vergl. z.B. DE-A 20 40 175; DE-A 21 19 518; Synthesis 1973, 703).

Als Verdünnungsmittel zur Durchführung des Herstellungsverfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutylketon; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester oder Basen wie Pyridin oder organische Säuren, wie Ameisensäure oder Essigsäure.

Das Herstellungsverfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Lithium-diethylamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, Lithiurn-organische Verbindungen, wie n-Butyllithium sowie tertiäre Amine, wie Trimethylamin, Triethylamin, Tributylamin, Di-isopropyl-ethylamin, Tetramethylguanidin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Alternativ kommen als Reaktionshilfsmittel auch organische oder anorganische Säuren, wie beispielsweise Schwefelsäure, Salzsäure, p-Toluolsulfonsäure, Perfluorbutansulfonsäure oder stark saure Ionenaustauscher infrage, besonders wenn in den verwendeten Alkylierungsmitteln der Formel (III) A für einen Hydroxy-, Acyloxyoder Alkoxyrest steht.

Das Herstellungverfahren kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-$C_{13}$/$C_{15}$-alkylammoniumchlorid, Trimethyl-$C_{13}$/$C_{15}$-alkylammoniumbromid, Dibenzyl-dimethyl-ammoniummethylsulfat, Dimethyl-$C_{12}$/$C_{14}$alkylbenzylammoniumchlorid, Dimethyl-$C_{12}$/$C_{14}$-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die Reaktionstemperaturen können bei der Durchführung des Herstellungsverfahrens in einem größeren Bereich variiert werden. Im allgemeinen arbeitet man bei Temperaturen zwischen -70°C und +200°C, vorzugsweise bei Temperaturen zwischen 0°C und 130°C.

Das Herstellungsverfahren wird üblicherweise unter Normaldruck durchgeführt. Es ist jedoch auch möglich unter erhöhtem oder vermindertem Druck zu arbeiten.

Zur Durchführung des Herstellungsverfahrens setzt man pro Mol an 1H-Benzimidazol der Formel (II) im allgemeinen 1,0 bis 5,0 Mol, vorzugsweise 1,0 bis 2,5 Mol an Alkylierungsmittel der Formel (III) und gegebenenfalls 0,01 bis 5,0 Mol, vorzugsweise 1,0 bis 3,0 Mol an Reaktionshilfsmittel ein.

In einer besonderen Durchführungsform ist es auch möglich, die 1H-Benzimidazole der Formel (II) zunächst in einem vorgelagerten Reaktionsschritt mit Hilfe üblicher Silylierungsverfahren beispielsweise mit Hexamethyldisilazan oder Trimethylsilylchlorid, gegebenenfalls in Gegenwart eines geeigneten Katalysators, wie beispielsweise Schwefelsäure, Trifluoressigsäure, Ammoniumsulfat, Imidazol oder Saccharin bei Temperaturen zwischen -20°C und +150°C zu silylieren und die so erhältlichen 1-Trimethylsilylbenzimidazole in einer anschließenden zweiten Stufe mit Alkylierungsmitteln der Formel (II) gemäß dem Herstellungverfahren umzusetzen. In diesem Fall ist es von Vorteil als Katalysator zur Alkylierungsreaktion Zinntetrachlorid zuzusetzen (vergl. z.B. Chem. Heterocycl. Comp. USSR 24, 514 [1988]).

Die Reaktionsdurchführung, Aufarbeitung und Isolierung der Reaktionsprodukte erfolgt nach bekannten Verfahren (vergl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen - insbesondere bei Regioisomerengemischen - mit Hilfe der Protonen-Kernresonanzspektroskopie ($^1$H-NMR).

Die Wirkstoffe eignen sich bei günstiger Warmblütertoxizität zur Bekämpfung von parasitischen Protozoen, die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Stämme wirksam. Durch die Bekämpfung der parasitischen Protozoen sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist.

Zu den parasitischen Protozoen zählen:

Mastigophora (Flagellata) wie z.B. Trypanosomatidae z.B. Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie z.B. Trichomonadidae z.B. Giardia lamblia, G. canis.

Sarcomastigophora (Rhizopoda) wie Entamoebidae z.B. Entamoeba histolytica, Hartmanellidae z.B. Acanthamoeba sp., Hartmanella sp.

Apicomplexa (Sporozoa) wie Eimeridae z.B. Eimeria acervulina, E. adenoides, E. alabahmensis, E. anatis, E. anseris, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana,E. leucarti, E. magna, E. maxima, E. media, E. meleagridis, E. meleagrimitis, E. mitis, E. necatrix, E. ninakohlyakimovae, E. ovis, E. parva,E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Neospara caninum, Cystisospora spec., Cryptosporidium spec. wie Toxoplasmadidae z.B. Toxoplasma gondii, wie Sarcocystidae z.B. Sarcocystis bovicanis, S. bovihominis, S. ovicanis, S. ovifelis, S. spec., S. suihominis wie Leucozoidae z.B. Leucozytozoon simondi, wie Plasmodiidae z.B. Plasmodium berghei, P. falciparum, P. malariae, P. ovale, P. vivax, P. spec., wie Piroplasmea z.B. Babesia argentina, B. bovis, B. canis, B. spec., Theileria parva, Theileria spec., wie Adeleina z. B. Hepatozoon canis, H. spec.

Ferner Myxospora und Microspora z.B. Glugea spec. Nosema spec.

Ferner Pneumocystis carinii, sowie Ciliophora (Ciliata) wie z.B. Balantidium coli, Ichthiophthirius spec., Trichodina spec., Epistylis spec.

Die erfindungsgemäßen Verbindungen sind auch wirksam gegen Protozoen, die als Parasiten bei Insekten auftreten. Als solche seien genannt Parasiten des Stammes Microsporida, insbesondere der Gattung Nosema. Besonders genannt sei Nosema apis bei der Honigbiene.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutzund Zierfische.

Zu Labor- und Versuchstieren gehören Mäuse, Ratten, Meerschweinchen, Goldhamster, Hunde und Katzen.

Zu den Hobbytieren gehören Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z.B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus cephalus), Pompano, Gilthread seabream (Sparus auratus), Tilapia spp., Chichliden-Arten wie z.B. Plagioscion, Channel catfish. Besonders geeignet sind die erfindungsgemäßen Mittel zur Behandlung von Fischbrut, z.B. Karpfen von 2 bis 4 cm Körperlänge. Sehr gut geeignet sind die Mittel auch in der Aalmast.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pouron and spot-on) und desEinpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspension zur oralen oder dermalen Anwendung sowie zur Injektion; Halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindem. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden, wie oben bei den Injektionslösungen beschrieben, hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden, wie oben bei den Injektionslösungen beschrieben, hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder von Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/-Caprinsäure-biglycerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter, eventuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$,

Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt:

nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenylpolyglykolether;

ampholytische Tenside wie Di-Na-N-lauryl-ß-iminodipropionat oder Lecithin;

anionaktive Tenside, wie Na-Laurylsulfat, Fettalkoholethersulfate, Mono/Dialkylpolyglykoletherorthophosphorsäureester-monoethanolaminsalz; kationaktive Tenside wie Cetyltrimethylammoniumchlorid.

Als weitere Hilfsstoffe seien genannt:

Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgerührt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen.

Besonders hervorgehoben seien Mischungen der erfindungsgemäßen Verbindungen mit einem Polyetherantibiotikum oder einem synthetisch hergestellten Coccidiosemittel.

Als synthetische Coccidiosemittel bzw. als Polyetherantibiotika zur Verwendung in den erfindungsgemäßen Mischungen seien bevorzugt genannt:

Amprolium, z.T. in Kombination mit Folsäureantagonisten
Robenidin
Toltrazuril
Monensin
Salinomycin
Maduramicin.

Besonders hervorgehoben sei die Mischung mit Maduramicin.

Anwendungsfertige Zubereitungen enthalten die Wirkstoffe in Konzentrationen von 10 ppm bis 20 Gewichtsprozent, bevorzugt von 0,1 bis 10 Gewichtsprozent.

Zubereitungen die vor Anwendung verdünnt werden, enthalten den Wirkstoff in Konzentrationen von 0,5 bis 90 Gewichtsprozent, bevorzugt von 1 bis 50 Gewichtsprozent.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

In der Mischung mit anderen Coccidiosemitteln oder Polyetherantibiotika liegen die erfmdungsgemäßen Wirkstoffe im Verhältnis 1 zu 0,1 - 10 bis 1 zu 1 - 10 vor. Bevorzugt ist das Verhältnis 1 zu 5.

Die Wirkstoffe können auch zusammen mit dem Futter oder Trinkwasser der Tiere verabreicht werden.

Futter- und Nahrungsmittel enthalten 0,01 bis 250 ppm, vorzugsweise 0,5 bis 100 ppm des Wirkstoffs in Kombination mit einem geeigneten eßbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Träger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Beispielhaft sei der Einsatz bei der Coccidiose genannt:

Für die Heilung und Prophylaxe etwa der Coccidiose bei Geflügel, insbesondere bei Hühnern, Enten, Gänsen und Truthähnen, werden 0,1 bis 100 ppm, vorzugsweise 0,5 bis 100 ppm eines Wirkstoffs mit einem geeigneten eßbaren Material, z.B. einem nahrhaften Futtermittel, gemischt. Falls gewünscht, können diese Mengen erhöht werden, besonders wenn der Wirkstoff vom Empfänger gut vertragen wird. Entsprechend kann die Verabreichung über das Trinkwasser erfolgen.

Für die Behandlung von Einzeltieren, z.B. im Falle der Behandlung der Coccidiose bei Säugetieren oder der Toxoplasmose, werden vorzugsweise Wirkstoffmengen von 0,5 bis 100 mg/kg Körpergewicht täglich verabreicht, um die gewünschten Ergebnisse zu erzielen. Trotzdem kann es zeitweilig notwendig sein, von den genannten Mengen abzuweichen, insbesondere in Abhängigkeit vom Körpergewicht des Versuchstieres oder der Art der Verabreichungsmethode, aber auch wegen der Tiergattung und seiner individuellen Reaktion auf den Wirkstoff oder der Art der Formulierung und der Zeit oder dem Abstand, zu dem er verabreicht wird. So kann es in gewissen Fällen genügen, mit weniger als der vorstehend genannten Mindestmenge auszukommen, während in anderen Fällen die genannte obere Grenze überschritten werden muß. Bei der Verabreichung größerer Mengen kann es zweckmäßig sein, diese im Verlauf des Tages in mehrere Einzeldarreichungen zu unterteilen.

Die Wirksamkeit der erfindungsgemäßen Verbindungen läßt sich z.B. in Käfigversuchen mit folgender Versuchsanordnung belegen, bei der die Tiere mit den jeweiligen Einzelkomponenten sowie mit den Mischungen der Einzelkomponenten behandelt werden.

Ein wirkstoffhaltiges Futter wird so zubereitet, daß die erforderliche Menge Wirkstoff mit einem nährstoffmäßig ausgeglichenen Tierfutter, z.B. mit dem unter angegebenen Kükenfutter, gründlich vermischt wird.

Wenn ein Konzentrat oder eine Vormischung zubereitet werden soll, die schließlich im Futter auf die im Versuch genannten Werte verdünnt werden soll, werden im allgemeinen etwa 1 bis 30 %, vorzugsweise etwa 10 bis 20 Gew.-% Wirkstoff mit einem eßbaren organischen oder anorganischen Träger, z.B. Mais- und Sojamehl oder Mineralsalzen, die eine kleine Menge eines eßbaren Entstäubungsöls, z.B. Maisöl oder Sojabohnenöl enthalten, vermischt. Die so erhaltene Vormischung kann dann dem vollständigen Geflügelfutter vor der Verabreichung zugegeben werden.

Als Beispiel für die Verwendung der erfindungsgemäßen Stoffe im Geflügelfutter kommt die folgende Zusammensetzung in Frage.

| | |
|---|---|
| 52,00 % | Futtergetreideschrot, und zwar: 40 % Mais, 12 % Weizen |
| 17,00 % | Sojaschrot extr. |
| 5,00 % | Maisklebefutter |
| 5,00 % | Weizenfuttermehl |
| 3,00 % | Fischmehl |
| 3,00 % | Mineralstoffmischung |
| 3,00 % | Luzernegrasgrümehl |
| 2,50 % | Vitaminvormischung |
| 2,00 % | Weizenkeime, zerkleinert |

(fortgesetzt)

| | |
|---|---|
| 2,00 % | Sojaöl |
| 2,00 % | Fleischknochenmehl |
| 1,50 % | Molkenpulver |
| 1,00 % | Melasse |
| 1,00 % | Bierhefe, gebunden an Biertreber |
| 100,00 % | |

Ein solches Futter enthält 18 % Rohprotein, 5 % Rohfaser, 1 % Ca, 0,7 % P sowie je kg 1200 i.E. Vitamin A, 1200 i.E. Vitamin D3, 10mg Vitamin E, 20mg Zinkbacitracin.

**Käfigversuch Coccidiose/Küken**

Coccidienfrei aufgezogene 8 bis 12 Tage alte männliche Hühnerküken (z.B. LSL Brinkschulte/Senden) erhalten von 3 Tage vor (Tag -3) der Infektion (= a.i.) bis 8 (9) Tage nach der Infektion (= p.i.) die erfindungsgemäßen Verbindungen (Testsubstanzen) in der in ppm angegebenen Konzentration mit dem Futter. In jedem Käfig werden 3 Tiere gehalten. Je Dosierung werden ein bis mehrere derartige Gruppen eingesetzt. Die Infektion erfolgt mittels einer Schlundsonde direkt in den Kropf mit etwa 100.000 sporulierten Oocysten von Eimeria acervulina sowie mit jeweils etwa 30.000 Oocysten von E. maxima und 40.000 sporulierten Oocysten von E. tenella. Es handelt sich hierbei um hochvirulente Stämme. Die genaue Infektionsdosis wird so eingestellt, daß möglichst eins von drei experimentell infizierten unbehandelten Küken infektionsbedingt stirbt. Für die Beurteilung der Wirksamkeit werden die folgenden Kriterien berücksichtigt: Gewichtszunahme von Versuchsbeginn bis Versuchsende, infektionsbedingte Sterberate, makroskopische Beurteilung der Faeces hinsichtlich Durchfall und Blutausscheidung an den Tagen 5 und 7 p.i. (Bewertung 0 bis 6), makroskopische Beurteilung der Darmschleimhaut, insbesondere der Blinddärme (Bewertung 0 bis 6) und die Oocystenausscheidung sowie der Anteil (in %) der innerhalb von 24 Stunden sporulierenden Oocysten. Die Zahl der Oocysten im Kot wurde mit Hilfe der McMaster-Zählkammer bestimmt (siehe Engelbrecht und Mitarbeiter "Parasitologische Arbeitsmethoden in Medizin und Veterinärmedizin", Akademie-Verlag, Berlin (1965)). Die einzelnen Befunde werden in Relation zu den unbehandelten nicht infizierten Kontroll-Gruppen gesetzt und eine Gesamtbewertung errechnet (vgl. A. Haberkorn (1986), S. 263 bis 270 in Research in Avian Coccidiosis ed L.R. McDougald, L.P. Joyner, P.L. Long, Proceedings of the Georgia Coccidiosis Conference, Nov. 18.-20.,1985 Athens/Georgia USA).

Versuchsergebnisse mit erfindungsgemäßen Kombinationen sind in den folgenden Tabellen beispielhaft aufgeführt. Die synergistische Wirksamkeit der Kombinationen im Vergleich zu den Einzelkomponenten wird besonders an der Reduktion der Oocystenausscheidung aber auch bezüglich der Sektionsbefünde, Gewichtsentwicklung und besseren Verträglichkeit ersichtlich.

In den folgenden Tabellen bedeutet in Spalte "Treatment" die Angabe

n.inf.contr. = nicht infizierte Kontrollgruppe
inf.contr. = infizierte Kontrollgruppe
1 = Benzimidazol Beispiel Nr.

In der Spalte "ppm" wird die eingesetzte Konzentration des Wirkstoffs im Futter in ppm angegeben.

In der Spalte "mortality" wird angegeben unter % der Prozentsatz der gestorbenen Tiere und unter n die Anzahl der gestorbenen Tiere/im Versuch eingesetzten Tiere.

In der Spalte "weight % of not inf. control" wird das Verhältnis des Gewichts der behandelten Tiere zum Gewicht der nicht infizierten Kontrollgruppe angegeben.

In den Spalten "dropping scores", "lesion score" und "oocyst control" werden Einzelangaben zur Wirkung gemacht.

In der Spalte "% efficacy" wird die Gesamtwertung bonitiert; 0 % bedeutet keine Wirkung, 100 % bedeutet volle Wirkung.

Tabelle 1

| Experimentelle Infektion mit Eimeria acervulina, Eimeria maxima und Eimeria tenella an Küken. Bsp. 1 in Kombination mit Monensin. | | | | | | | | | | | |
|---|---|---|---|---|---|---|---|---|---|---|---|
| Treatment | ppm | Mortality | | weight % of not inf. control | dropping scores | lesion score | oocyst in % of inf. control | | | | % efficacy tot. |
| | | % | n | | | | ac. | max. | ten. | tot. | |
| n.inf.contr. | 0 | 0 | 0/6 | 100 | 0 | 0 | 0 | 0 | 0 | 0 | 100 |
| inf.contr. | 0 | 100 | 6/6 | 0 | 6 | 6 | 100 | 100 | 100 | 100 | 0 |
| Bsp. 1 | 7,5 | 0 | 0/3 | 76 | 6 | 5,3 | 3 | 0 | 1 | 1,3 | 65 |
| Bsp. 1 | 10 | 0 | 0/3 | 84 | 6 | 3,3 | 0 | 0 | 1 | 0 | 75 |
| Mon. | 25 | 33 | 1/3 | 36 | 6 | 6 | 98 | 0 | 100 | 66 | 45 |
| Mon. | 50 | 33 | 1/3 | 81 | 6 | 6 | 44 | 0 | 13 | 19 | 36 |
| Mon. | 100 | 33 | 1/3 | 30 | 6 | 6 | 8 | 0 | 100 | 36 | 24 |
| Bsp. 1 + Mon. | 7,5 + 25 | 0 | 0/3 | 93 | 6 | 0 | 0 | 0 | 2 | 0,6 | 88 |
| Bsp. 1 + Mon. | 10 + 25 | 33 | 1/3 | 95 | 3 | 0,5 | 0 | 0 | 0 | 0 | 100 |
| Bsp. 1 + Mon. | 7,5 + 50 | 0 | 0/3 | 100 | 0 | 1,7 | 0 | 0 | 0 | 0 | 100 |
| Bsp. 1 + Mon. | 10 + 50 | 0 | 0/3 | 95 | 0 | 0,7 | 0 | 0 | 0 | 0 | 100 |
| Bsp. 1 + Mon. | 7,5 + 100 | 33 | 1/3 | 114 | 0 | 0 | 0 | 0 | 0 | 0 | 100 |
| Bsp. 1 + Mon. | 10 + 100 | 0 | 0/3 | 99 | 0 | 0 | 0 | 0 | 0 | 0 | 100 |

Tabelle 2

| Experimentelle Infektion mit Eimeria acervulina, Eimeria maxima und Eimeria tenella an Küken. Bsp. 1 in Kombination mit Maduramicin. | | | | | | | | | | | |
|---|---|---|---|---|---|---|---|---|---|---|---|
| Treatment | ppm | mortality | | weight % of not inf. control | dropping scores | lesion score | oocyst in % of inf. control | | | | % efficacy tot. |
| | | % | n | | | | ac. | max. | ten. | tot. | |
| n.inf.contr. | 0 | 0 | 0/6 | 100 | 0 | 0 | 0 | 0 | 0 | 0 | 100 |
| inf.contr. | 0 | 50 | 3/6 | 58 | 6 | 6 | 100 | 100 | 100 | 100 | 0 |

Tabelle 2 (fortgesetzt)

| Experimentelle Infektion mit Eimeria acervulina, Eimeria maxima und Eimeria tenella an Küken. Bsp. 1 in Kombination mit Maduramicin. | | | | | | | | | | | |
|---|---|---|---|---|---|---|---|---|---|---|---|
| Treatment | ppm | mortality | | weight % of not inf. control | dropping scores | lesion score | oocyst in % of inf. control | | | | % efficacy tot. |
| | | % | n | | | | ac. | max. | ten. | tot. | |
| Bsp. 1 | 1 | 33 | 1/3 | 31 | 6 | 6 | 49 | 100 | 97 | 82 | 36 |
| Bsp. 1 | 2,5 | 33 | 1/3 | 42 | 4-6 | 6 | 19 | 100 | 55 | 58 | 36 |
| Bsp. 1 | 5 | 0 | 0/3 | 110 | 1 | 0 | 1 | 100 | 11 1 | 37 | 90 |
| MAD | 1 | 0 | 0/3 | 93 | 1 | 2 | 8 | 100 | 30 | 46 | 73 |
| MAD | 2 | 0 | 0/3 | 114 | 0 | 2 | 8 | 100 | 45 | 51 | 84 |
| MAD | 3 | 0 | 0/3 | 112 | 0 | 0 | 1 | 0 | 3 | 1,3 | 100 |
| Bsp. 1 + MAD | 2,5 + 1 | 0 | 0/3 | 122 | 0 | 0 | 0 | 0 | 0 | 0 | 100 |
| Bsp. 1 + MAD | 5 + 1 | 0 | 0/3 | 119 | 0 | 0 | 0 | 0 | 0 | 0 | 100 |
| Bsp. 1 + MAD | 2,5 + 2 | 0 | 0/3 | 122 | 0 | 0 | 0 | 0 | 0 | 0 | 100 |
| Bsp. 1 + MAD | 5 + 2 | 0 | 0/3 | 105 | 0 | 0 | 0 | 0 | 0 | 0 | 100 |
| Bsp. 1 + MAD | 2,5 + 3 | 0 | 0/3 | 117 | 0 | 0 | 0 | 0 | 0 | 0 | 100 |
| Bsp. 1 + MAD | 1 + 3 | 0 | 0/3 | 121 | 0 | 0 | 0 | 0 | 0 | 0 | 100 |

Tabelle 3

| Experimentelle Infektion mit Eimeria acervulina, Eimeria maxima und Eimeria tenella an Küken. Bsp. 1 in Kombination mitSalinomycin. | | | | | | | | | | | |
|---|---|---|---|---|---|---|---|---|---|---|---|
| Treatment | ppm | Mortality | | weight % of not inf. control | dropping scores | lesion score | oocyst in % of inf. control | | | | % efficacy tot. |
| | | % | n | | | | ac. | max. | ten. | tot. | |
| n.inf.contr. | 0 | 0 | 0/6 | 100 | 0 | 0 | 0 | 0 | 0 | 0 | 100 |
| inf.contr. | 0 | 100 | 6/6 0 | 0 | 6 | 6 | 100 | 100 | 100 | 100 | 0 |
| Bsp. 1 | 5 | 0 | 0/3 | 72 | 6 | 6 | 6 | 0 | 30 | 12 | 45 |
| Bsp. 1 | 7,5 | 0 | 0/3 | 76 | 6 | 5,3 | 3 | 0 | 1 | 1,3 | 65 |

Tabelle 3 (fortgesetzt)

| Experimentelle Infektion mit Eimeria acervulina, Eimeria maxima und Eimeria tenella an Küken. Bsp. 1 in Kombination mitSalinomycin. | | | | | | | | | | | |
|---|---|---|---|---|---|---|---|---|---|---|---|
| Treatment | ppm | Mortality | | weight % of not inf. control | dropping scores | lesion score | oocyst in % of inf. control | | | | % efficacy tot. |
| | | % | n | | | | ac. | max. | ten. | tot. | |
| Bsp. 1 | 10 | 0 | 0/3 | 84 | 6 | 3,3 | 0 | 0 | 1 | 0 | 75 |
| Sal. | 15 | 100 | 3/3 | T | T | T | T | T | T | T | T |
| Sal. | 30 | 100 | 3/3 | T | T | T | T | T | T | T | T |
| Sal. | 60 | 0 | 0/3 | 106 | 6 | 1,3 | 6 | 0 | 31 | 12 | 67 |
| Bsp. 1 + Sal | 7,5 + 15 | 0 | 0/3 | 107 | 0 | 0 | 0 | 0 | 0 | 0 | 100 |
| Bsp. 1 + Sal | 10 + 15 | 33 | 1/3 | 103 | 0 | 0 | 0 | 0 | 0 | 0 | 100 |
| Bsp. 1 + Sal | 5 + 30 | 0 | 0/3 | 121 | 0 | 0 | 0 | 0 | 0 | 0 | 100 |
| Bsp. 1 + Sal | 10 + 30 | 33 | 1/3 | 103 | 0 | 0 | 0 | 0 | 0 | 0 | 100 |
| Bsp. I + Sal | 5 + 60 | 0 | 0/3 | 91 | 2 | 0 | 0 | 0 | 0 | 0 | 100 |
| Bsp. 1 + Sal | 10 + 60 | 33 | 1/3 | 97 | 0 | 0 | 0 | 0 | 0 | 0 | 100 |

In der folgenden Tabelle werden die Ergebnisse der Wirksamkeitsversuche mit den erfindungsgemäßen Verbindungen zusammengefaßt:

Tabelle 4

| Wirksamkeit gegen Eimeria acervulina, Eimeria maxima und Eimeria tenella | | | |
|---|---|---|---|
| Verbindung | Dosis in ppm | | |
| | 10 | 5 | 2,5 |
| Bsp. 1 | 222 | 222 | 111 |
| Bsp.2 | T T T | 222 | 121 |
| Bsp.3 | 222 | 222 | 021 |
| Bsp. 4 | 111 | 111 | 121 |
| Bsp. 5 | 112 | 111 | 121 |
| Bsp.6 | 222 | 222 | 111 |
| Bsp.7 | 221 | 222 | 212 |

Tabelle 4 (fortgesetzt)

| Wirksamkeit gegen Eimeria acervulina, Eimeria maxima und Eimeria tenella | | | |
|---|---|---|---|
| Verbindung | Dosis in ppm | | |
| | 10 | 5 | 2,5 |
| Bsp.8 | 222 | 011 | 000 |
| Bsp.9 | 222 | 221 | 011 |

Bewertungsschema:

2 = volle Wirkung
1 = schwache Wirkung
0 = unwirksam
T = Tod

Die folgenden Herstellbeispiele sollen die vorliegende Erfindung illustrieren aber nicht begrenzen:

**Beispiel 1**

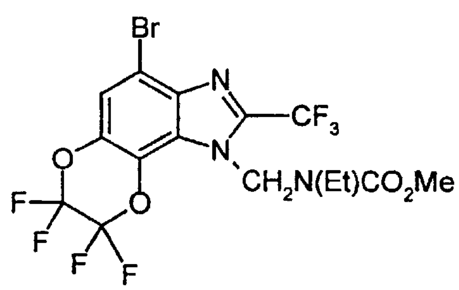

39,0 g (0,1 mol) 4-Brom-6,7-tetrafluor-6,7-dihydro-2-trifluormethyl-1H-[1,4]dioxino[2,3-e]benzimidazol legt man in 1 000 ml abs. $CH_2Cl_2$ vor, gibt 17,5 ml (0,125 mol) Triethylamin zu und tropft bei 20°C 18,9 g (0,125 mol) N-Chlormethyl-N-ethyl-carbamidsäuremethylester zu und refluxiert 24 h. Man wäscht zweimal mit je 250 ml Wasser und mit 250 ml gesättigter, wäßriger Kochsalzlösung und trocknet über Natriumsulfat. Der nach dem Eindampfen erhaltene Rückstand (54,4 g) wird an 1 kg Kieselgel (35-70 um) mit Cyclohexan/Ethylacetat (10:1) chromatographiert. Ausbeute: 30,6 g (60 % der Theorie), Fp: 92-93°C.

Analog zu Beispiel 1 und gemäß den allgemeinen Angaben zur Herstellung wurden die folgenden Beispiele 2-12 der Formel erhalten.

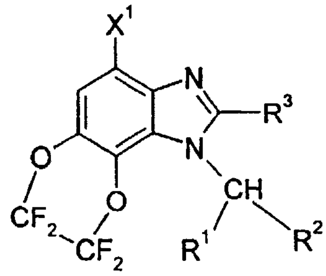

| Beispiel Nr. | $X^1$ | $R^1$ | $R^2$ | $R^3$ | Fp [°C] | $^1$H-NMR($CDCl_3$): δ [ppm] |
|---|---|---|---|---|---|---|
| 2 | Br | H | $CH_2N(Me)CO_2Me$ | $CF_3$ | | 2,8 (s, 3H),<br>6,07 (s, 2H) |
| 3 | Cl | H | $CH_2N(Me)CO_2Me$ | $CF_3$ | 72-74 | |
| 4 | Cl | H | $CH_2N(Et)CO_2Me$ | $CF_3$ | | 0,97 (t, 3H),<br>3,80 (s, 3H),<br>7,26 (s, 1H), |
| 5 | Br | H | $CH_2N(Bu)CO_2Et$ | $CF_3$ | | 0,76 (t, 3H),<br>3,06 (q, 2H),<br>6,08 (s, 2H), |
| 6 | Br | H | $CH_2N(iPr)CO_2Et$ | $CF_3$ | | 1,13 (d, 6H),<br>3,32 (Sept., 1H),<br>6,06 (s, 2H), |
| 7 | Cl | H | $CH_2N(iPr)CO_2Et$ | $CF_3$ | | 1,15 (d, 6H),<br>3,38 (sept., 1H)<br>6,12 (s, 2H) |
| 8 | Cl | H | $CH_2N(2\text{-}Me\text{-}C_6H_4)CO_2Me$ | $CF_3$ | 134-136 | |
| 9 | Cl | H | $CH_2N(C_6H_{11})CO_2Et$ | $CF_3$ | 85-88 | |
| 10 | Br | H | $CH_2N(2\text{-}Me\text{-}C_6H_4)CO_2Me$ | $CF_3$ | 135-136 | |
| 11 | Cl | H | $CH_2N(Pr)CO_2Et$ | $CF_3$ | | 0,77 (t, 3H)<br>4,23 (q, 2H)<br>6,14 (s, 2H) |

Me = Methyl
Bu = n-Butyl
Et = Ethyl
iPr = iso-Propyl
Pr = n-Propyl

Die Herstellung der Ausgangsverbindung für die Beispiele 1, 2, 6 und 8 kann, wie im folgenden angegeben, durchgeführt werden:

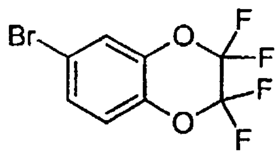

1 400 g (6,7 mol) 2,3-Tetrafluor-1,4-benzodioxan und 7 g (0,08 mol) FeS (Pulver) legt man vor, tropft bei 20 bis 30°C in ca. 4 h 1 190 g (7,4 mol) Brom zu und rührt ca. 20 h bis zum Ende der Gasentwicklung nach. Man wäscht mit wäßriger Natriumsulfitlösung und trocknet über Natriumsulfat. Der Rückstand wird im Vakuum destilliert.
Ausbeute: 1 540 g (80 % der Theorie), $Kp_{10}$: 70-74°C (GC: 99 %).

**Beispiel b)**

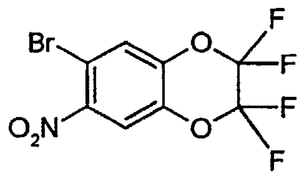

350 g (1,2 mol) 6-Brom-2,3-tetrafluor-1,4-benzodioxan tropft man bei 20°C in 75 min zu 273 ml (98 %ige) Salpetersäure und 293 ml konz. Schwefelsäure zu, rührt 1 h bei 20°C und 3 h bei 40°C nach. Man gießt den Ansatz auf Eis, extrahiert mit Methylenchlorid, wäscht die organische Phase mit Wasser, mit wäßriger Natriumhydrogencarbonatlösung und trocknet über Natriumsulfat. Die organische Phase wird eingedampft und als Rohprodukt weiter umgesetzt.

Ausbeute: 396 g (98 % d. Th.), (roh, GC: 99,1 %), $Kp_{16}$: 121-124°C, nD: 1,5065 bei 20°C.

**Beispiel c)**

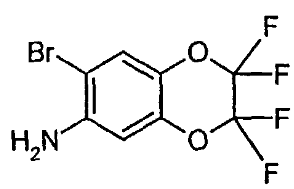

396 g (1,2 mol) 6-Brom-7-nitro-2,3-tetrafluor-1,4-benzodioxan legt man in 1 400 ml Ethanol vor, gibt 253 g (4,5 mol) Fe-Pulver zu und erhitzt zum Rückfluß. Dann tropft man unter Rückfluß 29 g konz. Salzsäure zu und rührt 1 h nach, tropft in der Siedehitze 43 ml Wasser zu und rührt 2 h nach. Der Ansatz wird abgekühlt, der Niederschlag abgesaugt und mit Ethanol gewaschen. Die Mutterlauge wird alkalisch gestellt und eingedampft. Der Rückstand wird in Methylenchlorid aufgenommen und zweimal mit Wasser gewaschen und über Natriumsulfat getrocknet. Die organische Phase wird eingedampft.

Ausbeute: 313 g (87 % d. Th.), (GC: 95,3 %).

**Beispiel d)**

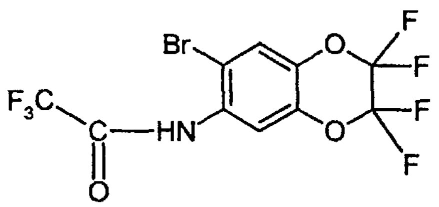

313 g (1,04 mol) 7-Amino-6-brom-2,3-tetrafluor-1,4-benzodioxan legt man in 1250 ml Toluol und 500 g (4,4 mol) Trifluoressigsäure vor und gibt portionsweise bei 20 bis 25°C 188 g (1,3 mol) Phosphorpentoxid zu. Der Ansatz wird klumpig. Man erhitzt 1 h auf 80°C (nicht rührbar). Man versetzt mit 500 ml Wasser und rührt noch 1 h bei 80°C nach. Nach Abkühlung wird die organische Phase abgetrennt und über Natriumsulfat getrocknet. Der eingedampfte Rückstand (340 g) enthält noch 50 % Edukt (GC).

Daher wird der Rückstand (340 g, 50 %ig) nochmals mit 1250 ml Toluol und 500 g (4,4 mol) Trifluoressigsäure versetzt, dann portionsweise 188 g (1,3 mol) Phosphorpentoxid zugegeben und 5 h auf 80°C erhitzt. Man dekantiert die organische Phase ab, wäscht diese zweimal mit Wasser, trocknet über Natriumsulfat und dampft ein.

Ausbeute: 273 g (66 % d. Th.), Fp.: 79 - 81°C (GC: 98 %)

Den klumpigen Reaktionsrückstand versetzt man mit Wasser, trennt den organischen Anteil ab, trocknet über Natriumsulfat und dampft ein.

Ausbeute: 56 g (14 %), (GC: 87 %).

**Beispiel e)**

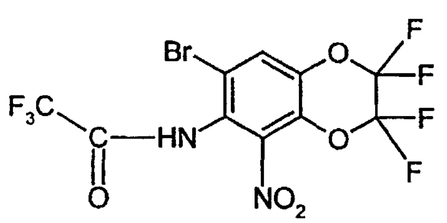

273 g (0,7 mol) 6-Brom-7-trifluormethylcarbonylamino-2,3-tetrafluor-1,4-benzodioxan legt man in 2047 ml konz. Schwefelsäure vor und tropft in 15 min bei 0°C 300 g Mischsäure zu und versetzt den dicken Brei bei 0 bis 20°C mit 1000 ml Methylenchlorid. Die Lösung wird dann bei 40°C 2 h nachgerührt. Man gießt den abgekühlten Kolbeninhalt auf Eiswasser und isoliert den Niederschlag. Die organische Phase wird aus der Mutterlauge abgetrennt und über Natriumsulfat getrocknet Der eingedampfte Rückstand und der isolierte Niederschlag werden vereinigt.

Ausbeute: 269 g (88 % d. Th.), Fp.: 158 - 159°C (GC: 100 %).

**Beispiel f)**

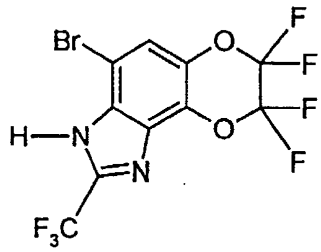

347 g (0,8 mol) 6-Brom-8-nitro-7-trifluormethylcarbonylamino-2,3-tetrafluor 1,4-benzodioxan legt man in 1735 ml Ethanol vor und gibt 183 g (3,3 mol) Fe-Späne und 183 g (3,3 mol) Fe-Pulver zu. Unter Rückfluß werden 38,5 ml Salzsäure zugetropft, 1 h nachgerührt, anschließend 58 ml Wasser zugetropft und 15 h refluxiert. Der abgekühlte Ansatz wird abgesaugt, die Mutterlauge alkalisch gestellt und eingedampft. Der Rückstand wird in Methylenchlorid aufgenommen, zweimal mit Wasser gewaschen, über Natriumsulfat getrocknet und eingedampft. Der Rückstand (170 g) wird an 1 kg Kieselgel (35 - 70 µm) mit Cyclohexan/Ethylacetat (5:1) chromatographiert.

Ausbeute: 125 g (40 % d. Th.), Fp. 162 - 164°C.

Die so erhältliche Verbindung des Beispiels f) kann gegebenenfalls ohne weitere Reinigung durch Alkylierung an den erfindungsgemäßen Verbindungen weiter umgesetzt werden.

**Patentansprüche**

1. Die Benzimidazole der Formel (1)

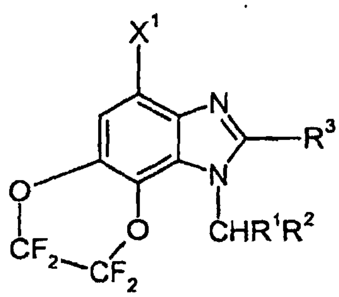

(I),

in welcher

$X^1$ für Chlor oder Brom steht,

$R^1$ Wasserstoffoder $C_{1-4}$-Alkyl steht,

$R^3$ für Fluoralkyl steht,

$R^2$ fiir den Rest

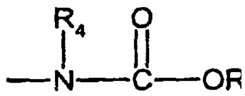

steht,

$R^4$ für Alkyl oder substituiertes Phenyl, das substituiert ist durch $C_{1-4}$-Alkyl, $C_{1-4}$-Halogenalkyl, Halogen, Nitro, $C_{1-4}$-Alkoxy, $C_{1-4}$-Halogenalkoxy, Methylendioxy, Ethylendioxy, die ihrerseits halogensubsituiert sein können, steht,

$R^5$ für Alkyl steht.

**2.** Verfahren zur Herstellung der Benzimidazole der Formel (1)

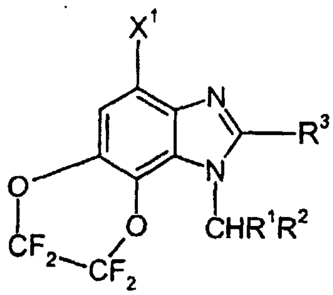

(I),

in welcher

$X^1$ für Chlor oder Brom steht,

$R^1$ Wasserstoff oder $C_{1-4}$-Alkyl steht,

$R^3$ für Fluoralkyl steht,

$R^2$ für den Rest

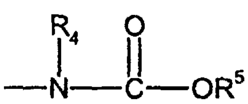

steht,

$R^4$ fiir Alkyl oder substituiertes Phenyl, das substituiert ist durch $C_{1-4}$-Alkyl, $C_{1-4}$-Halogenalkyl, Halogen, Nitro, $C_{1-4}$-Alkoxy, $C_{1-4}$-Halogenalkoxy, Methylendioxy, Ethylendioxy, die ihrerseits halogensubsituiert sein können, steht,

$R^5$ für Alkyl steht,

**dadurch gekennzeichnet, daß** man
1 H-Benzimidazole der Formel (II)

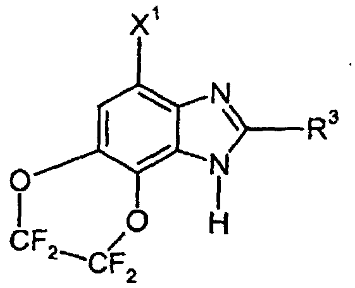

(II),

in welcher

$X^1$ und $R^3$ die oben angegebene Bedeutung haben,

mit einem Alkylierungsmittel der Formel (III)

$$A\text{-}CHR^1R^2 \quad (III),$$

in welcher

A für eine geeignete Abgangsgruppe steht,

$R^1$ und $R^2$ die oben angegebene Bedeutung haben,

gegebenenfalls in Gegenwart von Verdünnungsmitteln und/oder Reaktionshilfsmitteln umsetzt.

3. Mittel gegen parasitäre Protozoen, **gekennzeichnet durch** einen Gehalt an mindestens einem substituierten Benzimidazol der Formel (1) gemäß Anspruch 1.

4. Verwendung von substituierten Benzimidazolen der Formel (I) gemäß Anspruch 1 zur Herstellung von Mitteln gegen parasitäre Protozoen.

5. Mischungen von substituierten Benzimidazolen der Formel (I) gemäß Anspruch 1 mit Polyetherantibiotika oder synthetischen Coccidiosemitteln zur Bekämpfung von parasitären Protozoen.

## Claims

1. The benzimidazoles of the formula (I)

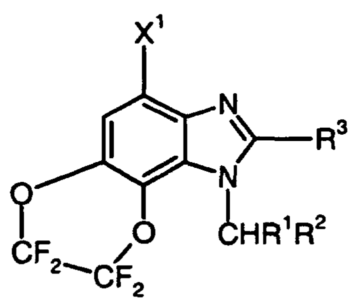

(I),

in which

$X^1$ represents chlorine or bromine,

$R^1$ represents hydrogen or $C_{1-4}$-alkyl,

$R^3$ represents fluoroalkyl,

$R^2$ represents the radical

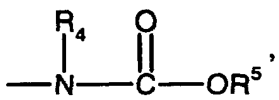

$R^4$ represents alkyl or substituted phenyl which is substituted by $C_{1-4}$-alkyl, $C_{1-4}$-halogenoalkyl, halogen, nitro, $C_{1-4}$-alkoxy, $C_{1-4}$-halogenoalkoxy, methylenedioxy, ethylenedioxy, which for their part can be halogen-substituted,

$R^5$ represents alkyl.

**2.** Process for the preparation of the benzimidazoles of the formula (I)

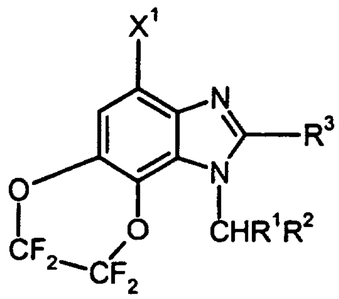

(I),

in which

$X^1$ represents chlorine or bromine

$R^1$ represents hydrogen or $C_{1-4}$-alkyl,

$R^3$ represents fluoroalkyl,

$R^2$ represents the radical

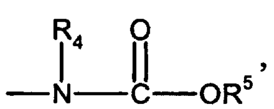

$R^4$ represents alkyl or substituted phenyl which is substituted by $C_{1-4}$-alkyl, $C_{1-4}$-halogenoalkyl, halogen, nitro, $C_{1-4}$-alkoxy, $C_{1-4}$-halogenoalkoxy, methylenedioxy, ethylenedioxy, which for their part can be halogen-substituted,

$R^5$ represents alkyl,

**characterized in that**
1 H-benzimidazoles of the formula (II)

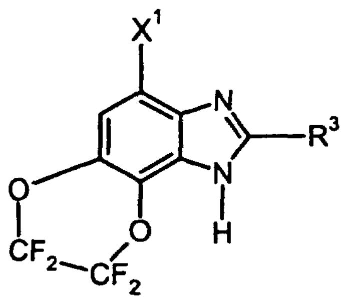

(II),

in which

$X^1$ and $R^3$ have the meaning indicated above,

are reacted with an alkylating agent of the formula (III)

$$A\text{-}CHR^1R^2 \qquad (III),$$

in which

A represents a suitable leaving group,

$R^1$ and $R^2$ have the meaning indicated above,

if appropriate in the presence of diluents and/or reaction auxiliaries.

3. Composition against parasitic protozoa, **characterized in that** it contains at least one substituted benzimidazole of the formula (I) according to Claim 1.

4. Use of substituted benzimidazoles of the formula (I) according to Claim 1 for the preparation of compositions against parasitic protozoa.

5. Mixtures of substituted benzimidazoles of the formula (I) according to Claim 1 with polyether antibiotics or synthetic coccidiosis agents for controlling parasitic protozoa.

**Revendications**

1. Benzimidazoles de la formule (I) :

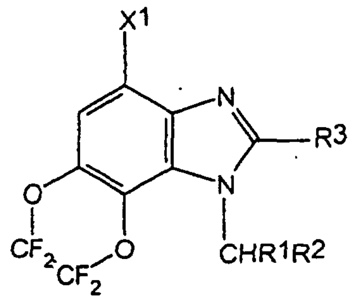

(I)

dans laquelle :

$X^1$ représente l'atome de chlore ou de brome ;
$R^1$ représente l'atome d'hydrogène ou un radical alcoyle en $C_{1-4}$ ;
$R^3$ représente un radical fluoroalcoyle ;
$R^2$ représente le reste $-N(R^4)-C(O)-OR^5$ ;
$R^4$ représente un radical alcoyle ou phényle substitué, qui est substitué par un radical alcoyle en $C_{1-4}$, halogénoalcoyle en $C_{1-4}$, un atome d'halogène, le radical nitro, un radical alcoxy en $C_{1-4}$, halogénoalcoxy en $C_{1-4}$, méthylènedioxy, éthylènedioxy, qui peuvent être pour leur part, substitués par un atome d'halogène ;
$R^5$ représente un radical alcoyle.

**2.** Procédé de préparation de benzimidazoles de la formule (I) :

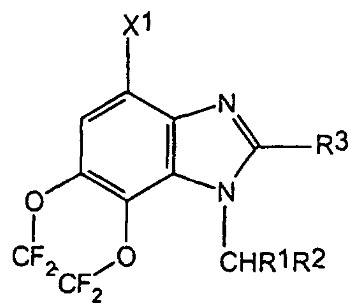

(I)

dans laquelle :

$X^1$ représente l'atome de chlore ou de brome ;
$R^1$ représente l'atome d'hydrogène ou un radical alcoyle en $C_{1-4}$ ;
$R^3$ représente un radical fluoroalcoyle ;
$R^2$ représente le reste $-N(R^4)-C(O)-OR^5$ ;
$R^4$ représente un radical alcoyle ou phényle substitué, qui est substitué par un radical alcoyle en $C_{1-4}$, halogénoalcoyle en $C_{1-4}$, un atome d'halogène, le radical nitro, un radical alcoxy en $C_{1-4}$, halogénoalcoxy en $C_{1-4}$, méthylènedioxy, éthylènedioxy, qui peuvent être pour leur part, substitués par un atome d'halogène ;
$R^5$ représente un radical alcoyle,

**caractérisé en ce que** l'on fait réagir des 1H-benzimidazoles de la formule (II) :

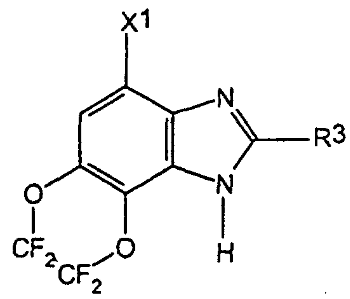

(II)

dans laquelle :

$X^1$ et $R^3$ ont la signification indiquée ci-dessus.

avec un agent d'alcoylation de la formule (III) :

$$A\text{-}CHR^1R^2 \quad \text{(III)}$$

dans laquelle:

A représente un groupe partant approprié,
$R^1$ et $R^2$ ont la signification indiquée ci-dessus,

le cas échéant en présence d'agents de dilution et/ou d'auxiliaires de réaction.

**3.** Agent contre les protozoaires parasitaires, **caractérisé par** une teneur en au moins un benzimidazole substitué de la formule (I) suivant la revendication 1.

**4.** Utilisation de benzimidazoles substitués de la formule (I) suivant la revendication 1, pour la préparation d'agents contre les protozoaires parasitaires.

**5.** Mélanges de benzimidazoles substitués de la formule (I) suivant la revendication 1, avec des antibiotiques polyéther ou des agents synthétiques contre la coccidiose pour lutter contre des protozoaires parasitaires.